# EUROPÄISCHE PATENTANMELDUNG

(11) **EP 2 829 255 A1**
(43) Veröffentlichungstag der Anmeldung: **28.01.2015**
(21) Anmeldenummer: 14002524.8
(22) Anmeldetag: 21.07.2014
(51) Int. Cl.: A61F 5/11

(54) **Vorrichtung zur Nagelkorrektur**

(30) Priorität: 23.07.2013 DE 102013012220
(71) Anmelder: 3TO GmbH, 82041 Deisenhofen (DE)
(72) Erfinder: Sutor, Norbert, 82041 Deisenhofen (DE); Sutor, Franziska, 80333 München (DE); Sutor, Johannes, 80339 München (DE)
(74) Vertreter: Müller, Bernhard

(57) **Zusammenfassung**

Es wird eine Vorrichtung zur Nagelkorrektur beschrieben, die ein auf die Oberfläche eines menschlichen Nagels (4) aufzuklebendes Haftelement (2) sowie mindestens ein, bevorzugt aus federhartem Draht bestehendes, Federelement (1) umfasst. Die Vorrichtung ist dadurch gekennzeichnet, dass das Federelement (1) mittels fest am Haftelement (2) angebrachten Aufnahmeeinrichtungen (3) formschlüssig mit diesem verbunden werden kann und durch, aus seiner elastischen Verformung beim Anbringen resultierende, Rückstellkräfte ein Biegemoment auf die Nagelplatte überträgt.

Wesentliche Vorteile der Vorrichtung bestehen in ihrer besonders einfachen und schnellen Anwendung bedingt durch die Einstückigkeit des zunächst spannungsfrei aufklebbaren Haftelementes. Die Ausübung einer kontinuierlich wirkenden Federkraft auf den Nagel ohne invasiven Eingriff unter die Nagelränder erweist sich im Hinblick auf den angestrebten Heilungserfolg als besonders wirksam.

## Beschreibung

Die Erfindung betrifft eine Vorrichtung zur Nagelkorrektur gemäß Oberbegriff des Hauptanspruchs.

Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese so genannten Orthonyxiespangen reduzieren die Krümmung der Nagelplatte und entlasten dadurch den oftmals entzündeten Nagelfalz. Die Schmerzen werden deutlich gemindert und eine Abheilung ermöglicht.

Die Wirkung dieser Nagelkorrekturspangen beruht auf Rückstellkräften, außermittigen Zugkräften oder auf Kombinationen dieser beiden Mechanismen.

Folgende Vorrichtungen zur Nagelkorrektur sind bisher bekannt und werden zur Behandlung eingesetzt. Sie weisen jedoch entweder bezüglich ihrer Wirksamkeit oder ihrer Anwendung einige Nachteile auf:

### Drahtspangen zur Nagelkorrektur

Teilweise bereits seit Jahrzehnten werden unterschiedliche Drahtspangen zur Nagelkorrektur verwendet. Diese Spangen umgreifen mit Drahthaken die Nagelränder und ziehen diese leicht nach oben. Bekannt unter den Drahtspangen ist die sogenannte Fraserspange, eine einteilige Drahtspange, welche den Nagel mittels Rückstellkraft anhebt. Diese Spange wird vom Therapeuten für jeden Patienten individuell an einem, zuvor anhand eines Abdruckes herzustellenden, Modell angefertigt, da die Vorrichtung von vorne auf den Nagel aufgeschoben wird, was eine exakte Anpassung auf die Nagelbreite erfordert.

Eine Weiterentwicklung der Fraserspange stellen mehrteilige Drahtspangen dar, welche aus zwei mit Häkchen versehenen Schenkeln und einer in der Mitte anzubringenden Drahtschlaufe bestehen (siehe z.B. DE 42 07 797 A1) oder aus zwei Schenkeln aufgebaut sind (DE 10 2005 03 9147 B3). Der Therapeut versieht auch die teilweise vorgefertigten Spangenschenkel mit Häkchen und formt die Spange entsprechend der Nagelkrümmung und -dicke.

Die Anwendung der Drahtspangen erfordert großes Geschick des Therapeuten und macht eine spezielle Schulung unumgänglich. Zudem muss die Spange stets für den jeweiligen Nagel angepasst werden, was einen erheblichen Zeitaufwand zur Folge hat.

Die in den oft engen oder gar entzündeten Nagelfalz eingebrachten Häkchen stellen zudem ein erhebliches Verletzungsrisiko dar.

### Klebespangen zur Nagelkorrektur

Auf die Rückstellkraft von Metall- oder Kunststoffelementen basierende Klebespangen, wie beispielsweise die in der DE 37 08 811 A1 beschriebene und als "BS-Spange^{R}" vertriebene, Nagelkorrekturvorrichtung, werden im vorgespannten Zustand auf die Nagelplatte aufgeklebt. Dafür muss die Spange an den Nagel angedrückt werden, was bei ohnehin schmerzhaft eingewachsenen Nägeln mit erheblichen Schmerzen für den Patienten verbunden sein kann. Bei zu geringem Anpressdruck hingegen ist die Spange nicht ausreichend fixiert und kann sich wieder lösen. Auf Grund des steifen Materials ist die Anwendung bei sehr stark gekrümmten Nägeln oft gar nicht möglich.

Eine aufklebbare Nagelkorrekturspange mit der Möglichkeit der spannungsfreien Anbringung und anschließender stufenloser Aktivierung ist in der DE 10 2006 010 299 A1 beschrieben. Mit dieser als "podofix^{R}-Spange" bekannten Vorrichtung werden die Nagelränder durch Verdrillen des eingebetteten Aktivierungsdrahtes auf eine gewünschte Position eingestellt und in dieser gehalten. Dadurch wird keine weitere Korrektur der Nagelkrümmung erreicht, was jedoch für den Therapieerfolg meist von großer Bedeutung ist.

### Einseitig einhängbare Federspange mit aufklebbarem Haftelement

Eine einseitig unter den Nagelrand greifende Nagelkorrekturvorrichtung, welche auf der anderen Seite mittels Klebung auf dem Nagel befestigt wird, ist in der DE 10 2008 039 762 A1 beschrieben und ist als "COMBIped^{R}-Spange" am Markt bekannt. Diese Nagelkorrekturvorrichtung stellt eine Kombination aus Draht- und Klebespange dar und vereint eine schnelle und einfache Montage auf dem Nagel mit einer konstanten, dauerhaften Zugkraft, welche die Krümmung des Nagels kontinuierlich korrigiert. Bei manchen Nägeln ist jedoch die Zugänglichkeit des Nagelrandes auf beiden Seiten so stark eingeschränkt, dass kein Häkchen eingehängt werden kann. In diesem Fall ist das Anbringen dieser Spange nicht oder.nur mit erheblichem Verletzungsrisiko möglich.

### Nagelkorrekturspange bestehend aus zwei einzelnen Befestigungselementen und einem losen Federelement

Eine Vorrichtung bestehend aus zwei, auf den Nagel aufgeklebten Befestigungselementen, welche durch ein loses Federelement miteinander verbunden werden, ist in der DE 3330813 A1 beschrieben. Hier werden die Elemente zur Krafteinleitung im ungespannten Zustand auf die Nagelplatte geklebt, die Spangenkraft wird anschließend über ein einzuschiebendes Federelement aufgebracht.

Aufgrund der filigranen Abmessungen ist es für den Anwender schwierig, die Befestigungselemente einzeln in gemeinsamer Ausrichtung auf den Nagel aufzukleben und das Federelement anschließend in die vorgesehenen Aussparungen zu verbringen. Dies stellt hohe Ansprüche an das Geschick des Anwenders und bedeutet einen erhöhten Zeitaufwand für die einzelne Montage der zwei Befestigungselemente.

Aufgabe der vorliegenden Erfindung ist es, eine Vorrichtung zur Nagelkorrektur zu schaffen, welche einfach und schnell anwendbar ist und gleichzeitig eine kontinuierlich wirkende Kraft auf den Nagel erzeugt, ohne dass ein Häkchen unter den Nagel eingehängt werden muss.

Im Laufe von Entwicklungsarbeiten im Rahmen der vorliegenden Erfindung wurde festgestellt, dass es von besonderem Vorteil ist, wenn eine mittels Federkraft wirkende Nagelkorrekturvorrichtung zunächst spannungsfrei in einem Stück auf den Nagel aufgeklebt werden kann und erst nach dem Fixieren durch Spannen eines Federelementes aktiviert wird.

Der Gegenstand der Erfindung ist im Anspruch 1 definiert.

Fortentwicklungen und besondere Ausführungsformen der Erfindung ergeben sich aus den Unteransprüchen.

Nachstehend wird die Erfindung beispielhaft anhand einiger Zeichnungen näher erläutert:

Dabei zeigen:
Fig. 1 eine erfindungsgemäße Vorrichtung zur Nagelkorrektur im fertig auf dem Nagel montierten Zustand;
Figg. 2 und 3 die Vorrichtung gemäß Fig. 1 im ungespannten Lieferzustand in Seitenansicht bzw. Draufsicht;
Fig. 4 eine erfindungsgemäße Vorrichtung zur Nagelkorrektur im ungespannten Lieferzustand in einer zweiteiligen Ausführungsform;
Figg. 5 und 6 alternative Ausführungsformen zur Fixierung des Federelementes an den Befestigungseinrichtungen des Haftelements;
Figg. 7 bis 9 Ausführungsformen mit Manipulationshilfen zur leichteren Handhabung der Vorrichtung zur Nagelkorrektur.

Nachstehend wird auf die in den Figuren dargestellten Ausführungsformen näher eingegangen.

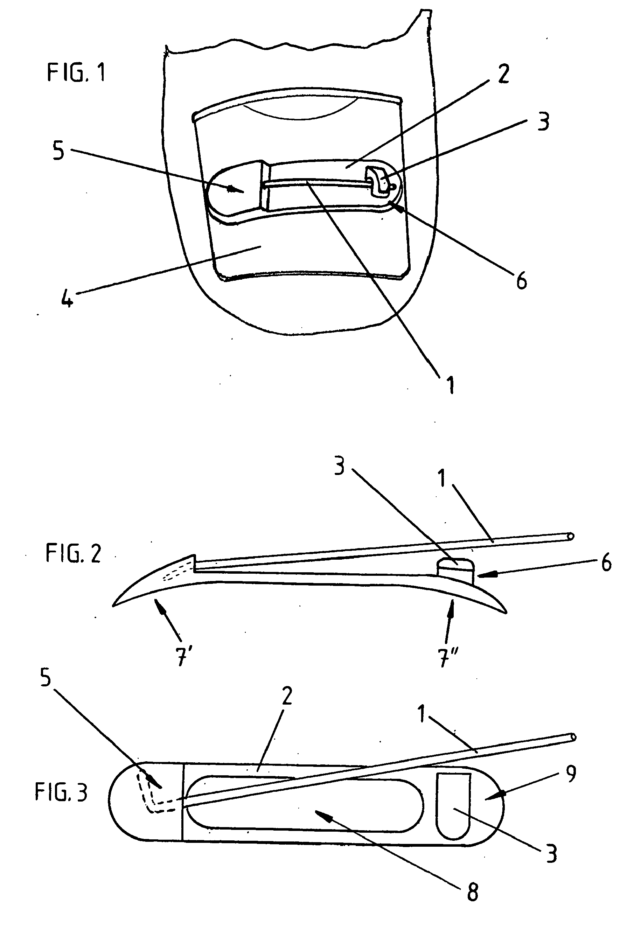

Figur 1 zeigt die erfindungsgemäße Vorrichtung im auf den Nagel montierten Zustand. Zur Behandlung des eingewachsenen Nagels 4 einer menschlichen Zehe sollen die Nagelränder minimal angehoben werden, um den schmerzenden Nagelfalz zu entlasten. Hierfür ist ein Federelement 1 dergestalt mit einem auf den Nagel aufgeklebten Haftelement 2 verbunden, dass durch die Rückstellkraft des ausgelenkten Federelementes 1 ein Biegemoment auf den Nagel 4 wirkt, welches die Nagelränder nach oben zieht.

Das Federelement 1 ist in der dargestellten Ausführung an der Befestigungsstelle 5 bereits fest mit dem Haftelement 2 verbunden. Die komplette Vorrichtung zur Nagelkorrektur kann somit in einem Arbeitsschritt auf den Nagel aufgebracht werden. Gegenüber der Befestigungsstelle 5 weist das Haftelement 2 eine Einrichtung zur formschlüssigen Aufnahme des Federelementes (Aufnahmeeinrichtung) 3 auf, hier in Form einer hakenförmigen Hinterschneidung 6. Dies nimmt das, in der dargestellten Ausführung aus federhartem Draht bestehende, Federelement 1 auf, nachdem das Haftelement 2 zuvor mittels eines schnell aushärtenden Klebstoffes zunächst spannungsfrei auf der Oberfläche des Nagels 4 fixiert wurde. Der Draht des Federelementes 1 wird dabei elastisch verformt und erzeugt somit eine Rückstellkraft auf das Haftelement 2 und damit auf den Nagel 4.

Die Intensität dieser Rückstellkraft kann vom Anwender der erfindungsgemäßen Vorrichtung zur Nagelkorrektur durch mehr oder minder starke Vorkrümmung des Federelements 1 und die Auswahl unterschiedlicher Materialquerschnitte desselben beeinflusst werden.

Figur 2 zeigt die erfindungsgemäße Vorrichtung im unmontierten Zustand der Auslieferung in der Seitenansicht, Figur 3 in der Draufsicht.

Das Haftelement 2 besteht in einer bevorzugten Ausführung aus einem zähelastischen Kunststoff. Für eine sichere Verklebung ist die Unterseite des Haftelementes 2 in ihren Randbereichen 7', 7", welche die wichtigsten Auflageflächen zur Verklebung mit der Oberfläche des Nagels darstellen, bereits im Lieferzustand vorgekrümmt.

In der hier dargestellten Ausführung weist das Haftelement 2 im mittleren Bereich eine Aussparung 8 auf, in welcher das Federelement 1 nach der Montage zu liegen kommt, was eine besonders flache Ausgestaltung der Vorrichtung zur Nagelkorrektur ermöglicht.

Das Federelement 1 besteht in der hier dargestellten Ausführungsform aus einem geraden, federharten Edelstahldraht, welcher an der Befestigungsstelle 5 fest in das Haftelement 2 eingebettet ist. Das drahtförmige Federelement 1 ist in einer bevorzugten Ausführungsform schräg zur Längsachse des Haftelementes 2 in der, der Öffnung der Aufnahmeeinrichtung 3 entgegengesetzten Richtung angeordnet, so dass der Draht 1 nach zum Einhängen leicht ausgelenkt werden muss und anschließend auf Grund seiner Federkraft sicher in der Hinterschneidung 6 verbleibt. Dies bietet den Vorteil, dass die Verbindung wieder gelöst werden kann und die Spannkraft der erfindungsgemäßen Vorrichtung bei Bedarf auch nachträglich durch plastisches Verbiegen des Federelementes 1 entgegen der Nagelkrümmung erhöht werden kann.

Das Federelement 1 kann, wie in Figuren 2 und 3 dargestellt, zunächst deutlich über die Aufnahmeeinrichtung 3 hinausragen und nach dem Einhängen in selbige mittels eines Seitenschneiders gekürzt werden. Hierzu weist das Haftelement 2 einen in Richtung Nagelrand über die Aufnahmeeinrichtung 3 hinausragenden, flach auf dem Nagel aufliegenden Bereich 9 auf, wodurch ein bündiger Schnitt entlang des Hakens ermöglicht und gleichzeitig die Hautpartie des Nagelfalzes vor unerwünschtem Stechen durch überstehende Drahtteile geschützt wird.

Zur endgültigen Fixierung des Federelementes 1 in der Aufnahmeeinrichtung 3 und zum Schutz des Patienten kann eine Versiegelung mit einem Klebstoff oder einer aushärtenden Kunststoffmasse vorgenommen werden.

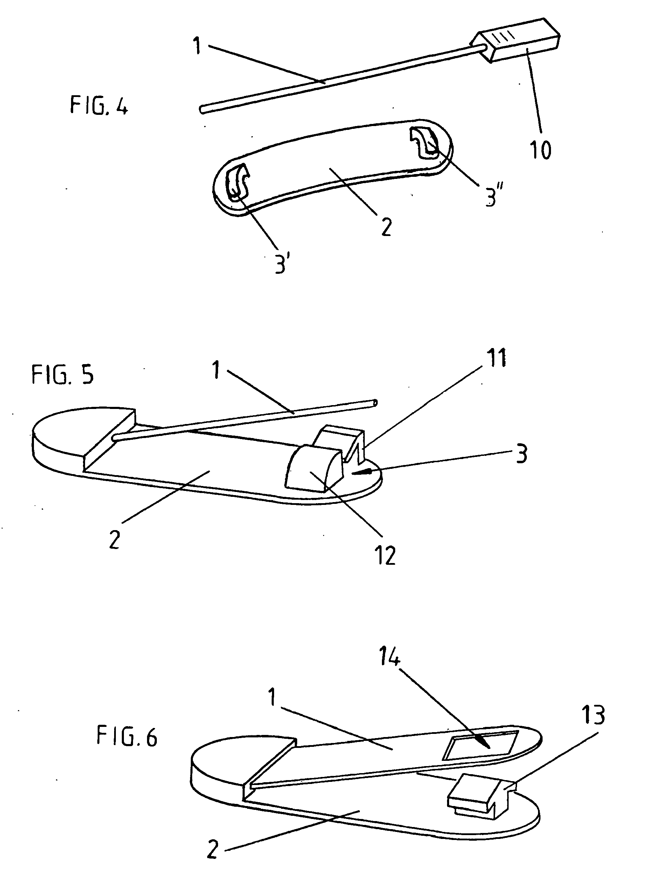

Figur 4 zeigt eine zweigeteilte Ausführungsform der erfindungsgemäßen Vorrichtung zur Nagelkorrektur im Lieferzustand. Das Federelement 1 ist bei dieser Ausführung nicht fest mit dem Haftelement 2 verbunden. Zum Aktivieren der Nagelkorrekturvorrichtung wird das Federelement 1 mittels mehrerer Aufnahmeeinrichtungen 3', 3'' nach dem Aufkleben mit dem Haftelement 2 verbunden. Zum einfacheren Einführen des Federelementes 1 in die Aufnahmeeinrichtungen 3', 3'' kann das Federelement 1 beispielsweise mit einem Haltegriff 10 versehen sein. Diese zweiteilige Ausführung bietet die Möglichkeit, je nach Kraftbedarf ein Federelement mit der für den aktuellen Therapiebedarf entsprechenden Stärke anzuwenden.

Bei der in Figur 5 dargestellten Ausführungsform der erfindungsgemäßen Vorrichtung zur Nagelkorrektur wird das als Draht ausgebildete Federelement 1 von einer als Widerhaken ausgebildeten Aufnahmeeinrichtung 3 am Haftelement 2 befestigt. Das Federelement 1 kann von oben zwischen den flexibel auf dem Haftelement 2 ausgebildeten Haken 11 und ein feststehendes Gegenstück 12 eingedrückt werden und wird nach dem Rückfedern des Hakens 11 in dessen Hinterschneidung formschlüssig fixiert. Das Eindrücken des Federelementes von Oben ist für den Anwender vor allem bei stark gekrümmten Nägeln einfacher als ein Einfädeln in eine seitliche Hinterschneidung.

Die in Figur 6 dargestellte erfindungsgemäße Vorrichtung zur Nagelkorrektur weist ein Federelement 1 auf, welches als Blattfeder ausgeführt ist. Die Einrichtung zur formschlüssigen Aufnahme des Federelementes 1 ist hier als ein, auf dem Haftelement 2 befindlicher, pfeil- oder pilzförmiger Zapfen 13 ausgebildet. Das flache Federelement 1 ist mit einer Aussparung 14 versehen, welche über den Zapfen 13 gedrückt werden kann, wodurch es am Haftelement 2 fixiert wird. Die Aussparung 14 ist länglich ausgebildet um Längendifferenzen bei unterschiedlichen Nagelkrümmungen ausgleichen zu können.

Das als Blattfeder ausgebildete Federelement 1 kann aus federhartem Metall aber auch aus rigidem Kunststoff bestehen.

Alternativ zu einer Blattfeder kann das Federelement 1 auch in Gestalt eines Drahtbügels ausgeführt sein, welcher den Zapfen 13 nach dem Aufdrücken umschließt.

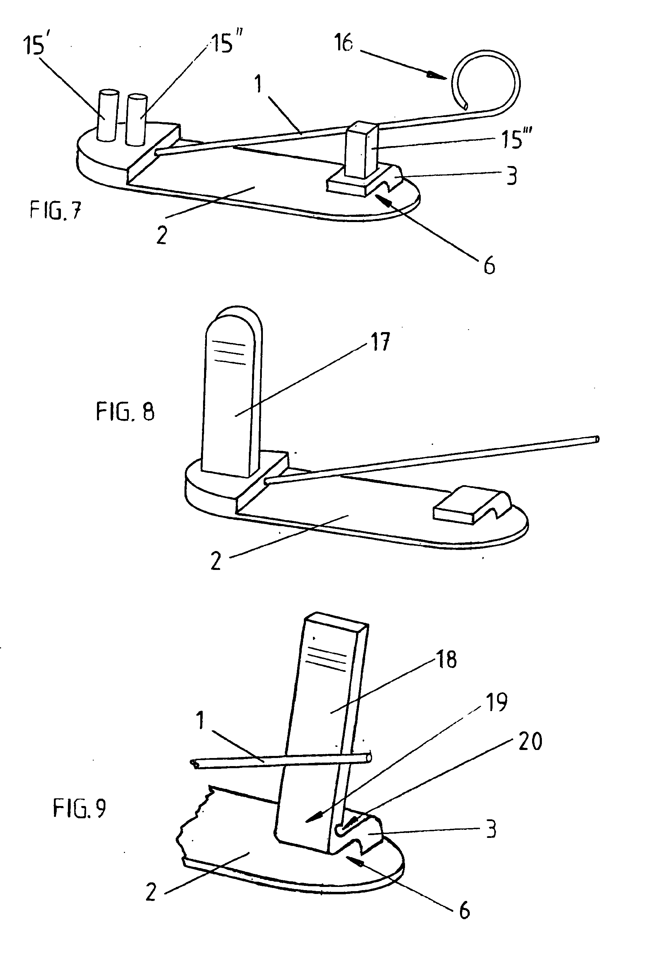

Figur 7 zeigt die erfindungsgemäße Vorrichtung zur Nagelkorrektur in einer mit Manipulationshilfen zur Handhabung mit Werkzeugen versehenen Ausführung. Hier weist das Haftelement 2 auf der Oberseite Zapfen 15`-15''' auf, welche mit einer Zange oder einer Pinzette gefasst werden können und damit die Handhabung sowohl beim Klebstoffauftrag als auch beim Aufsetzen auf den Nagel einfacher gestalten. Es können beispielsweise zwei runde Zapfen 15', 15'' oder ein rechteckiger Zapfen 15''' oder auch alternative Ansatzflächen für entsprechende Werkzeuge an der Vorrichtung vorhanden sein. Die Zapfen 15'-15''' werden nach dem Aufkleben der Vorrichtung zur Nagelkorrektur abgeschnitten, sodass die Vorrichtung zur Nagelkorrektur nach der Montage eine ebene Oberfläche aufweist.

Der über die Halteeinrichtung 3 hinaus stehende Rest des Federelementes 1 kann zum erleichterten Einführen in die Hinterschneidung 6 der Halteeinrichtung 3 beispielsweise mit einer Öse 16 versehen sein, welche nach der Montage abgetrennt wird.

In Figur 8 ist die erfindungsgemäße Vorrichtung zur Nagelkorrektur in einer Ausführungsform dargestellt, welche ein vereinfachtes, werkzeugloses Anbringen der Vorrichtung auf dem Nagel ermöglicht. Der auf dem Haftelement 2 angebrachte Griff 17 dient zum Greifen mit den Fingern, um das Aufkleben des Haftelementes 2 zu vereinfachen. Es können ein Griff an einem beliebigen Ende des Haftelementes oder auch zwei Griffe zum Fassen der Vorrichtung mit beiden Händen angebracht sein.

Der Griff 17 wird nach dem Anbringen der Vorrichtung zur Nagelkorrektur mit einem Seitenschneider, einer Schere oder einem Skalpell vom Haftelement 2 abgetrennt, so dass die Vorrichtung zur Nagelkorrektur im montierten Zustand eine glatte Oberfläche aufweist.

Figur 9 zeigt eine auf dem Haftelement 2 befindliche Aufnahmeeinrichtung 3, bei welcher ein Griff 18 so ausgestaltet ist, dass er als werkzeuglose Manipulationshilfe beim Aufkleben des Haftelementes auf den Nagel und gleichzeitig als Einführhilfe für das als Federdraht ausgebildete Federelement 1 in die Hinterschneidung 6 dient. Die Vorderseite des Griffes 18 bildet eine glatte Fläche 19 welche senkrecht in der Hinterschneidung 6 mündet. Auf der Rückseite weist der Griff 18 eine Mulde 20 auf, welche den Materialquerschnitt soweit reduziert, dass der gesamte Griff 18 nach vorne geneigt werden kann, ohne das aufgeklebte Haftelement 3 zu bewegen. Der zunächst weiter oben an der Fläche 19 des Griffes 18 anliegende Draht des Federelementes 1 gleitet beim Neigen des Griffes um 90° nach vorne in die Hinterschneidung 6 der Halteeinrichtung 3. Die Aktivierung der Vorrichtung zur Nagelkorrektur wird so weiter vereinfacht.

Wenn auf das Kippen des Griffes verzichtet werden soll, kann alternativ der Draht des Federelementes 1 mit einem stabförmigen Werkzeug oder dem Fingernagel entlang der Anlagefläche 19 in die Hinterschneidung 6 geschoben werden. Auch dies stellt eine Vereinfachung gegenüber dem freien Einhängen des Federelementes dar.

### Vorteile der erfindungsgemäßen Vorrichtung zur Nagelkorrektur

Gegenüber den bisher bekannten, herkömmlichen Verfahren und Vorrichtungen weist die erfindungsgemäße Vorrichtung zur Nagelkorrektur einige Vorteile auf.

Durch ihre schnelle und einfache Anwendung wird diese Nagelkorrekturspange der Anforderung der Wirtschaftlichkeit in der ärztlichen, podologischen und kosmetischen Praxis besonders gerecht.

Durch die Verbindung eines durch elastische Deformation aktivierbaren Federelementes mit einem auf die Nageloberfläche aufklebbaren Haftelement können eingewachsene und deformierte Nägel mit stetig wirkender Zugkraft behandelt werden, was sich für den Therapieerfolg als besonders günstig erwiesen hat. Durch das zunächst spannungsfreie Aufkleben des Haftelementes können auch derart stark verformte Nägel therapiert werden, auf welchen bereits vorgespannte Nagelkorrektureinrichtungen nicht befestigt werden könnten.

Die Einstückigkeit des Haftelementes verringert gegenüber Vorrichtungen mit zwei einzelnen Haftelementen die Montagezeit auf die Hälfte und gewährleistet gleichzeitig bedingt durch die größere Klebefläche einen besonders sicheren Halt auf der Nageloberfläche.

Sowohl das Anbringen als auch die anschließend erfolgende Aktivierung der Vorrichtung zur Nagelkorrektur kann auch von Laien ohne Probleme durchgeführt werden. Besonders bei den oben beschriebenen Ausführungen mit Manipulationshilfen ist eine völlig werkzeuglose Montage möglich.

Die besonders an der Nagelmitte sehr flache Bauweise dieser Vorrichtung zur Nagelkorrektur bietet dem Patienten einen hohen Tragekomfort. Sie wird auch in engen Schuhen nicht als störend empfunden.

Da die erfindungsgemäße Vorrichtung lediglich auf der Nageloberfläche angebracht wird, besteht bei der Anwendung keinerlei Verletzungs- oder Infektionsgefahr für den Patienten. Dies ist besonders bei Diabetikern und Blutern von großer Bedeutung.

## Patentansprüche

1. Vorrichtung zur Nagelkorrektur, umfassend
- ein auf die Nageloberfläche aufzuklebendes Haftelement,
- mindestens ein Federelement,
- mindestens eine Einrichtung zur Aufnahme eines Federelements,
**dadurch gekennzeichnet, dass** das oder die Einrichtungen zur Aufnahme eines Federelements fest mit dem Haftelement verbunden sind.

2. Vorrichtung nach Anspruch 1, **dadurch gekennzeichnet, dass** mindestens ein Federelement fest mit dem Haftelement verbunden ist.

3. Vorrichtung nach Anspruch 1 oder 2, **dadurch gekennzeichnet, dass** das Haftelement aus flexiblem Kunststoff besteht.

4. Vorrichtung nach einem der Ansprüche 1 bis 3, **dadurch gekennzeichnet, dass** das Federelement aus federhartem Draht besteht.

5. Vorrichtung nach einem der Ansprüche 1 bis 3, **dadurch gekennzeichnet, dass** das Federelement als Blattfeder aus Metall oder Kunststoff ausgebildet ist.

6. Vorrichtung nach einem der Ansprüche 1 bis 5, **dadurch gekennzeichnet, dass** an der Vorrichtung Einrichtungen zur Manipulationshilfe angebracht sind, die das Aufkleben des Haftelements und/oder das Anbringen des Federelements erleichtern.
